# EUROPEAN PATENT SPECIFICATION

(11) **EP 3 202 326 B1**
(45) Date of publication and mention of the grant of the patent: **04.12.2019**
(21) Application number: 15847218.3
(22) Date of filing: 02.10.2015
(51) Int. Cl.: A61B 8/00, G01S 7/52

(54) **ULTRASONIC PROBE ATTACHMENT, ULTRASONIC PROBE, ULTRASONIC INSPECTION APPARATUS AND ULTRASONIC INSPECTION METHOD**
ULTRASCHALLSONDENANBAU, ULTRASCHALLSONDE, ULTRASCHALLPRÜFVORRICHTUNG UND ULTRASCHALLPRÜFVERFAHREN
ATTACHE DE SONDE ULTRASONORE, SONDE ULTRASONORE, APPAREIL D'INSPECTION ULTRASONORE ET PROCÉDÉ D'INSPECTION ULTRASONORE

(30) Priority: 02.10.2014 JP 2014204136
(43) Date of publication of application: 09.08.2017
(73) Proprietor: Samsung Electronics Co., Ltd., Gyeonggi-do 16677 (KR)
(72) Inventor: IIJIMA, Kazuo, Yokohama-shi, Kanagawa 230-0027 (JP)
(74) Representative: Gulde & Partner
(86) International application number: PCT/KR2015/010438
(87) International publication number: WO 2016/053048

(56) References cited:
- CN-A- 104 068 892
- JP-A- H05 220 156
- JP-A- 2007 014 606
- JP-A- 2011 004 807
- JP-A- 2013 085 565
- US-A- 5 997 481
- US-A1- 2014 180 116

## Description

### TECHNICAL FIELD

The present invention relates to an attachment for ultrasound probe, an ultrasound probe, an ultrasound inspecting apparatus, and an ultrasound inspecting method.

### BACKGROUND ART

Ultrasound inspecting apparatuses including an ultrasound probe are widely used for medical and industrial purposes. Patent reference 1 (Japanese Patent Application Publication No. 2012-176197) discloses the use of a film-shaped echo gel for acoustic coupling between a probe and a human body when an ultrasound inspecting apparatus performs an ultrasound diagnosis. Furthermore, US 2014/0180116 A1 and US 5,997,481, too, disclose ultrasound inspecting apparatuses.

### DETAILED DESCRIPTION OF THE INVENTION

### TECHNICAL PROBLEM

The film-shaped echo gel has high ultrasonic transmission and is very flexible, thus leaving no sticky feeling after being peeled off. However, the film-shaped echo gel follows the irregular surface of the human body too closely, and thus a minute air layer may be formed between the probe and the echo gel. As a result, a jelly material for acoustic coupling between the probe and the human body by filling the air layer should be coated.

To address this problem, the present invention provides an attachment for ultrasound probe that does not form an air layer between a probe and an inspection target and does not need a jelly material, and an ultrasound probe, an ultrasound inspecting apparatus, and an ultrasound inspecting method.

### TECHNICAL SOLUTION

According to the present invention, an attachment for an ultrasound probe is provided, the attachment comprising: an acoustic matching element provided on a transceiver of the ultrasound probe and deformed based on a surface of an inspection target when the ultrasound probe scans the surface of the inspection target; a medium supplier provided near the acoustic matching element and configured to provide a medium between the inspection target and the acoustic matching element, based on the deformation of the acoustic matching element; and a body configured to fix the acoustic matching element and the medium supplier to the ultrasound probe; wherein the body accommodates the acoustic matching element and the medium supplier and has an opening in a surface thereof facing the inspection target, portions of the acoustic matching element and the medium supplier protrude from the opening; characterized in that the medium supplier surrounds at least some of lateral surfaces of the protruded portion of the acoustic matching element, wherein the lateral surfaces are surfaces of the acoustic matching element meeting with the surface of the acoustic matching element facing the inspection target. Preferably, the acoustic matching element has a convex shape having a protrusion, and the protrusion protrudes from the opening. Preferably, the opening has a groove that ranges from an inner side of the body to an outer side of the body and serves as a flow path of the medium. Preferably, the body further comprises a deformable fixer that is between the ultrasound probe and the body and fixes the ultrasound probe to the body. Preferably, the acoustic matching element has a hydrophilic property, and the medium is liquid including water. Preferably, the acoustic matching element comprises polymer gel. Preferably, the acoustic matching element is a water bag that accommodates water. Preferably, the medium is water or hyaluronic acid. Preferably, the medium supplier is pressed due to the deformation of the acoustic matching element and supplies the medium. Preferably, the medium supplier is in contact with the acoustic matching element and is directly pressed by the acoustic matching element due to the deformation of the acoustic matching element. Preferably, the medium supplier includes non-woven fabric. Preferably, the attachment may further comprise a supply increaser that comprises a pressure plate which is coupled to the body via a hinge and is pushable to press the medium supplier. Preferably, the attachment may further comprise a supply increaser that comprises a pressure plate which is coupled to the body via a hinge and is pushable to press the medium supplier.

According to the present invention, an ultrasound inspecting apparatus is provided, characterized in that the ultrasound inspecting apparatus comprises an attachment for ultrasound probes; and an ultrasound probe on which the attachment for ultrasound probes is mounted.

According to the present invention, an ultrasound inspecting method is provided, characterized in that the method comprises fixing an acoustic matching element and a medium supplier onto an ultrasound probe by using a body, the body having an opening in a surface thereof facing the inspection target, portions of the acoustic matching element and the medium supplier protruding from the opening, and the acoustic matching element being deformed based on a surface of an inspection target when the ultrasound probe scans the surface of the inspection target; surrounding at least some of lateral surfaces of the protruded portion of the acoustic matching element with a medium supplier, wherein the lateral surfaces are surfaces of the acoustic matching element meeting with the surface of the acoustic matching element facing the inspection target; supplying a medium between the inspection target and the acoustic matching element, based on the deformation of the acoustic matching element, wherein the supplying is performed by the medium supplier; and transmitting ultrasound waves to the inspection target and receiving reflected waves of the ultrasound waves from the inspection target, wherein the transmitting and the receiving are performed by the ultrasound probe.

### ADVANTAGEOUS EFFECTS

Disclosed embodiments provide an attachment for ultrasound probe that does not form an air layer between a probe and an inspection target and does not need a jelly material, an ultrasound probe, an ultrasound inspecting apparatus, and an ultrasound inspecting method.

### DESCRIPTION OF THE DRAWINGS

FIG. 1 illustrates an exterior of an attachment according to embodiment 1.
FIG. 2 illustrates exteriors of the attachment and a probe according to embodiment 1.
FIG. 3 is a cross-sectional view illustrating a schematic structure of the attachment according to embodiment 1 mounted on the probe.
FIG. 4 is a schematic cross-sectional view illustrating the probe according to embodiment 1 ultrasound-inspecting an inspection target by sending ultrasound waves to the inspection target.
FIG. 5 is a flowchart of a method in which the probe according to embodiment 1 ultrasound-inspects the inspection target.
FIG. 6 is an exploded perspective view for explaining a method of assembling the attachment according to embodiment 1.
FIG. 7 illustrates an acoustic matching element applicable to the attachment according to embodiment 1, according to another example.
FIG. 8 illustrates a medium supplier applicable to the attachment according to embodiment 1, according to another example.
FIGS. 9-11 are views for explaining a method of assembling another example of the attachment according to embodiment 1.
FIG. 12 illustrates a body of the attachment according to embodiment 1, according to another example.
FIG. 13 illustrates the exterior of an attachment according to embodiment 2.
FIG. 14 is a cross-sectional view illustrating a schematic structure of the attachment according to embodiment 2 mounted on a probe.
FIG. 15 illustrates the exterior of a probe according to embodiment 3.
FIG. 16 is a cross-sectional view illustrating a schematic structure of the probe according to embodiment 3.

### MODE OF THE INVENTION

An attachment for ultrasound probe, an ultrasound probe, an apparatus for inspecting ultrasound, and a method of inspecting ultrasound will now be described in detail with reference to the attached drawings. Like reference numerals in the drawings denote like elements, and, in the drawings, the sizes of elements may be exaggerated for clarity and for convenience of explanation. It will be understood that when a material layer is referred to as being "formed on" a substrate or another layer, it can be directly or indirectly formed on the substrate or the other layer. That is, for example, intervening layers may be present. Materials that constitute each layer in embodiments below are exemplary, and thus the other materials may be used. It will be understood that when a layer is referred to as being "on" another layer or substrate, it can be directly on the other layer or substrate, or intervening layers may also be present.

### <Embodiment 1>

An attachment for ultrasound probe according to the present embodiment 1 will now be described with reference to the attached drawings.

The attachment according to the present embodiment 1 is mounted on an ultrasound probe of an ultrasound inspecting apparatus. The attachment includes at least an acoustic matching element and a medium supplier. When the probe scans an inspection target, such as a human body, the acoustic matching element is located between the probe and the inspection target and secures acoustic matching and acoustic coupling between the probe and the inspection target, and the medium supplier supplies a medium between the acoustic matching element and the inspection target to secure wettability and lubricity between the probe and the inspection target. Accordingly, an ultrasound inspection may be smoothly conducted.

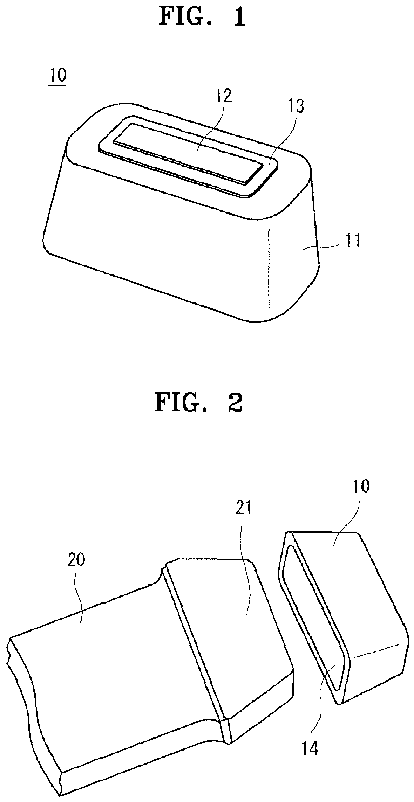

FIG. 1 illustrates the exterior of an attachment 10 according to the present embodiment 1. FIG. 1 illustrates the exterior of the attachment 10 when a surface of the attachment 10 that faces an inspection target faces upwards. The attachment 10 includes a body 11, an acoustic matching element 12 partially exposed via an approximately center portion of a surface of the body 11 facing the inspection target, and a medium supplier 13 partially exposed via a portion of the surface of the body 11 around the acoustic matching element 12.

The body 11 is used to fix the acoustic matching element 12 and the medium supplier 13 onto a probe, and may be formed of, for example, ABS (Acrylonitrile, Butadiene, Styrene) resin. Alternatively, the body 11 may be formed of a rigid paper material and thus may be crushed and downsized after being used. Moreover, the body 11 formed of a rigid paper material does not generate much poisonous gas when being incinerated, and thus may reduce medical waste.

The acoustic matching element 12 is used to reduce an acoustic mismatch caused due to a difference between acoustic impedances of the probe and the inspection target, and to secure acoustic coupling between the probe and the inspection target, and may be formed of, for example, polymer gel. The medium supplier 13 is used to hold a medium and supply the medium between the inspection target and the acoustic matching element 12, and may be formed of, for example, non-woven fabric. The medium maintains a surface of the acoustic matching element 12 in a wet condition to thereby form a lubrication space between the acoustic matching element 12 and the inspection target.

FIG. 2 illustrates the exteriors of the attachment 10 and a probe 20 according to the present embodiment 1. FIG. 2 illustrates the exteriors of the attachment 10 and the probe 20 when the attachment 10 and the probe 20 are viewed from the side. The attachment 10 has an opening 14 on a side of the body 11 opposite to the side thereof facing the inspection target, namely, on the side of the probe 20. The probe 20 is inserted into the attachment 10 via the opening 14 and thus the attachment 10 is fixed to the probe 20.

An ultrasound inspecting apparatus according to the present embodiment 1 includes the probe 20 and an image processing unit (not shown) to which the probe 20 is connected, and is used for various purposes, such as medical treatments and industrial applications. The probe 20 includes, on a leading end thereof, a transceiver 21 that transmits ultrasound waves and receives reflected waves of the transmitted ultrasound waves. The probe 20 may be a conventional probe. The image processing unit to which the probe 20 is connected processes a signal from the probe 20 to generate and display an ultrasound image that represents the internal status of the inspection target. Since the image processing unit may be a conventional image processing unit, illustration and descriptions thereof will be omitted.

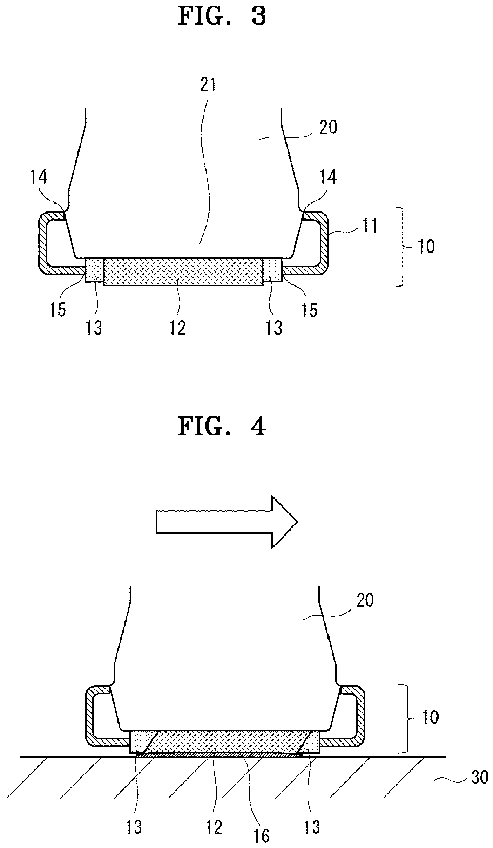

FIG. 3 is a cross-sectional view illustrating a schematic structure of the attachment 10 according to the present embodiment 1 mounted on the probe 20. FIG. 3 illustrates a schematic structure of the attachment 10 when the surface of the attachment 10 facing the inspection target faces down and the attachment 10 mounted on the probe 20 is viewed from the side.

The body 11 of the attachment 10 accommodates the acoustic matching element 12 and the medium supplier 13, and portions of the acoustic matching element 12 and the medium supplier 13 protrude and are exposed via an opening 15 formed in the surface of the body 11 facing the inspection target. The probe 20 is inserted into the opening 14 of the body 11 of the attachment 10, and thus the attachment 10 is mounted on the probe 20. At this time, the acoustic matching element 12 is disposed on (below in FIG. 3) the transceiver 21 of the probe 20.

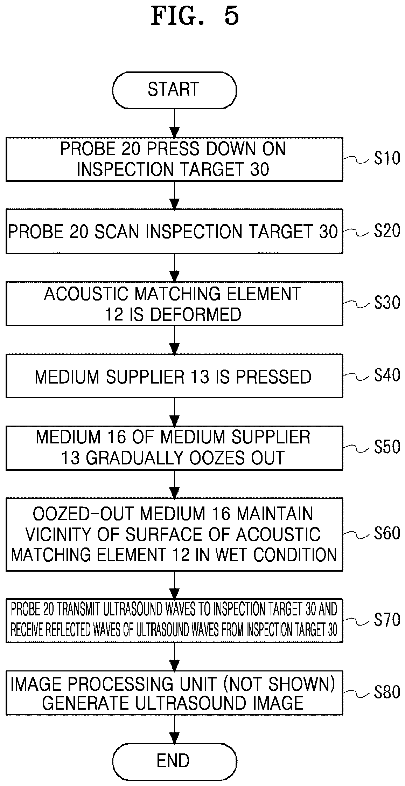

FIG. 4 is a schematic cross-sectional view illustrating the probe 20 according to the present embodiment 1 ultrasound-inspecting an inspection target 30 by scanning the inspection target 30. FIG. 5 is a flowchart of a method in which the probe 20 according to the present embodiment 1 ultrasound-inspects the inspection target 30.

First, when an ultrasound inspection starts, the probe 20 equipped with the attachment 10 presses down on the inspection target 30, in operation S10. In operation S20, the probe 20 scan the inspection target 30 from the left to the right in FIG. 4.

At this time, the acoustic matching element 12 contacts a surface of the inspection target 30 via a medium 16 or the like and is deformed based on the surface of the inspection target 30, in operation S30. Due to the deformation of the acoustic matching element 12, the medium supplier 13 is pressed by the acoustic matching element 12, in operation S40, and the medium 16 included in the medium supplier 13 gradually oozes out, in operation S50. In operation S60, the oozed-out medium 16 maintains the vicinity of the surface of the acoustic matching element 12 in a wet condition or a lubricated condition and reduces a friction between the acoustic matching element 12 and the inspection target 30. The probe 20 transmits ultrasound waves to the inspection target 30 and receives reflected waves of the ultrasound waves from the inspection target 30, in operation S70, and the image processing unit (not shown) generates an ultrasound image, in operation S80.

The medium supplier 13 is pressed by the deformed acoustic matching element 12 rather than by the surface of the inspection target 30. Accordingly, even when the probe 20 strongly presses down and scans the surface of the inspection target 30, a force applied to the medium supplier 13 is limited, and the amount of the medium 16 oozing out of the medium supplier 13 seldom increases. The medium supplier 13 may stably supply the medium 16 between the acoustic matching element 12 and the surface of the inspection target 30.

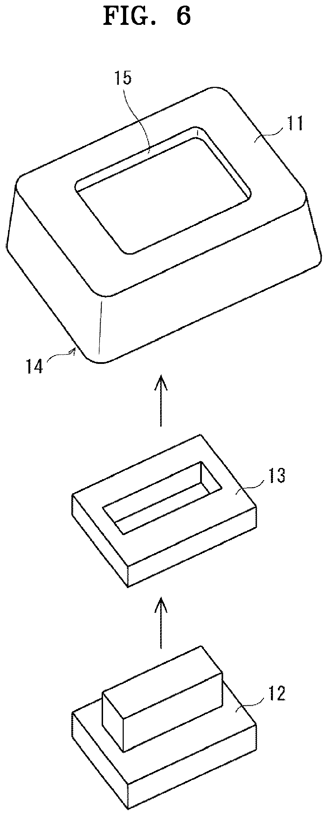

FIG. 6 is an exploded perspective view for explaining a method of assembling the attachment 10 according to the present embodiment 1. FIG. 6 illustrates the surface of the attachment 10 contacting an inspection target. The attachment 10 may be assembled with the body 11, the acoustic matching element 12, and the medium supplier 13.

The body 11 may be formed of the aforementioned ABS resin or the like. The body 11 has the opening 15 in the surface facing the inspection target, and the opening 14 in the surface opposite to the surface facing the inspection target, namely, in the surface on the side via which the probe 20 is inserted.

The acoustic matching element 12 may be formed of the aforementioned polymer gel, or may be formed of hydrogel having a hydrophilic property by a hydrophilic group, such as a hydroxyl group. The acoustic matching element 12 may have a convex shape or a flange shape. The convex shape of the acoustic matching element 12 may be obtained by cutting both sides out of a polymer gel sheet, or by fusing or adhering polymer gel corresponding to a protrusion onto the polymer gel sheet, or by molding polymer gel.

When a soft material, such as polymer gel, is used to form the acoustic matching element 12, the surface of the inspection target is prevented from being deformed by being pressed by the probe 20, and thus a natural and clear ultrasound image of a structure of the vicinity of the surface of the inspection target may be obtained.

By using the hydrophilic acoustic matching element 12 and a medium including water, an extremely small amount of medium may maintain the surface of the acoustic matching element 12 and its vicinity in a wet condition. Since the supplied medium is extremely small, the medium is immediately evaporated from the surface of the inspection target after the probe 20 passes the surface of the inspection target, and a to-be-inspected person is not given much unpleasant feeling. An operation of cleaning the medium from the surface of the inspection target is not necessary, in contrast with when a jelly material is used.

The medium supplier 13 is formed of the aforementioned non-woven fabric, such as a material that includes the medium and is capable of oozing out the medium, and has a 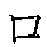 -character shape having a rectangular opening or a ⊐-character shape having a rectangular opening of which one side is open. The material used to form the medium supplier 13 may be a fabric such as pulp, rayon or polyester which is used in a sheet mask. The medium supplier 13 may be formed of sponge.

The medium supplier 13 may have a 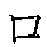 -character shape, a ⊐-character shape, or the like, and may surround at least some, preferably, two opposing lateral surfaces, of the lateral surfaces of the protrusion of the acoustic matching element 12, namely, the surfaces of the acoustic matching element 12 meeting with the surface of the acoustic matching element 12 facing the inspection target. In order for the medium supplier 13 to have a ⊐-character shape, two medium suppliers 13 may be combined.

The attachment 10 is assembled by pushing the medium supplier 13 and the acoustic matching element 12 into the body 11 via the opening 14 of the body 11 in this stated order such that at least the protrusion of the acoustic matching element 12 protrudes from the opening 15 of the body 11. At this time, the medium supplier 13 and the acoustic matching element 12 are pushed into the body 11 such that a portion of the medium supplier 13 also protrudes from the opening 15 of the body 11. The acoustic matching element 12 and the medium supplier 13 may directly contact each other or may be adjacent to each other via a thin film or the like. The important thing is that the attachment 10 is assembled such that the medium supplier 13 allows the medium to ooze out by being pressed by the acoustic matching element 12 when the acoustic matching element 12 has been deformed due to scanning. By forming the acoustic matching element 12 to have a convex shape, shoulder portions of the acoustic matching element 12, which are defined by the protrusion of the acoustic matching element 12, are caught by the opening 15 of the body 11, and thus the positions of the acoustic matching element 12 and the medium supplier 13 within the body 11 are stabilized.

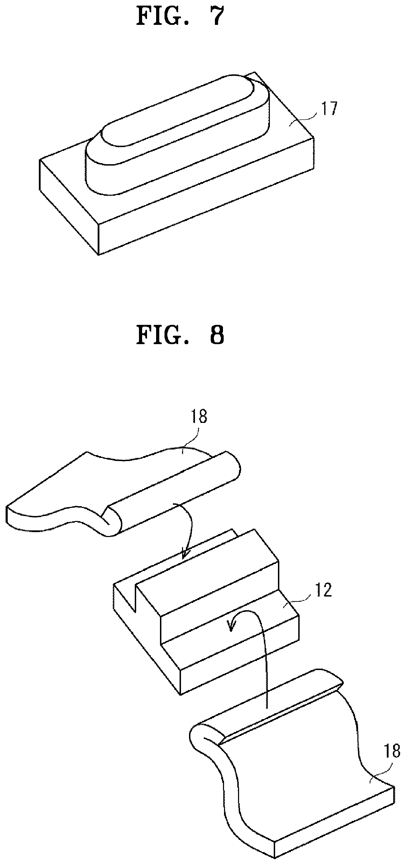

FIG. 7 illustrates an acoustic matching element 17, which is another example of the acoustic matching element 12 according to the present embodiment 1. Referring to FIG. 7, a protrusion of the acoustic matching element 17 may have an approximately oval shape by rounding a surface of the protrusion facing the inspection target. The edges of the protrusion may be chamfered. By having this shape, the acoustic matching element 17 may smoothly scan the surface of the inspection target. Moreover, the acoustic matching element 17 may have a shape that coincides with the shape of an acoustic lens of the probe 20.

FIG. 8 illustrates a medium supplier 18, which is another example of the medium supplier 13 according to the present embodiment 1. Referring to FIG. 8, the medium supplier 18 is divided into two sheets, each of which one end is rolled. The medium supplier 18 and the acoustic matching element 12 are pushed into the body 11 such that a rolled portion of each sheet is put on the shoulder portions of the acoustic matching element 12. In this case, the rolled portion of the medium supplier 18 may protrude from the opening 15 of the body 11.

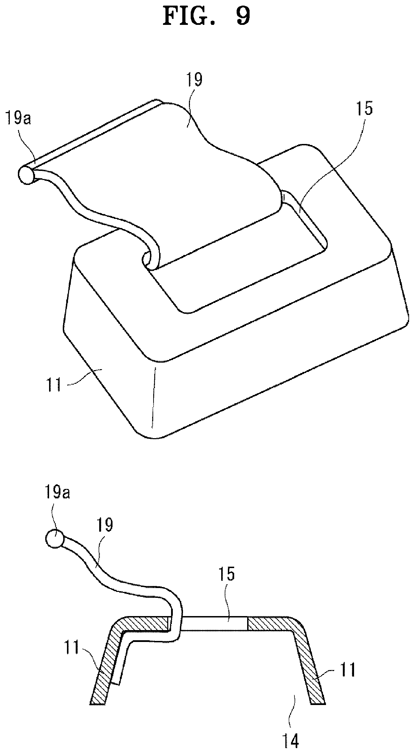

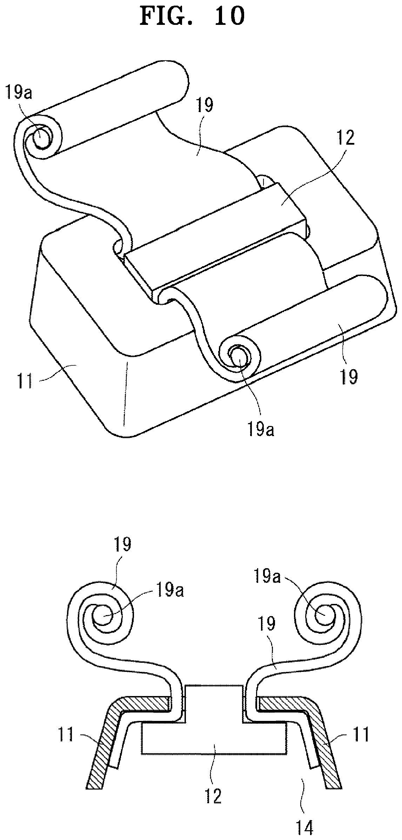

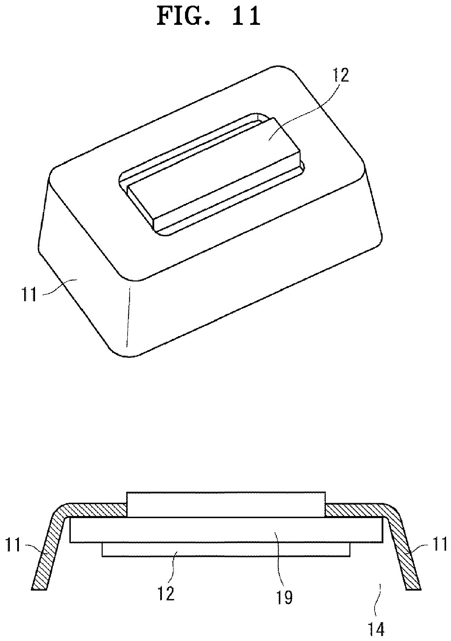

FIGS. 9-11 are views for explaining a method of assembling another example of the attachment 10 according to the present embodiment 1. FIGS. 9-11 illustrate the surface of the attachment 10 contacting an inspection target. The respective upper views of FIGS. 9-11 illustrate the exteriors of the attachment 10 in the assembling processes of the method. The respective lower views of FIGS. 9 and 10 illustrate cross-sections of the attachment 10 in a direction parallel to a shorter side of the surface of the attachment 10 facing the inspection target, and the lower view of FIG. 11 illustrates a cross-section of the attachment 10 in a direction parallel to a longer side of the surface of the attachment 10 facing the inspection target.

The attachment 10 is assembled with the body 11, the acoustic matching element 12, and a medium supplier 19. The medium supplier 19 has a sheet shape and has, at one end thereof, a core portion 19a in a rod shape formed of the same material as or a different material from the medium supplier 19.

First, as shown in FIG. 9, the other end of the medium supplier 19 previously rendered wet by a medium, which has no core portions 19a, is inserted into the body 11 via the opening 15. This operation is conducted on two sheets of medium suppliers 19. At this time, because the inserted medium supplier 19 is in a wet condition, the inserted medium supplier 19 adheres to an inner wall of the body 11.

Next, as shown in FIG. 10, the acoustic matching element 12 is inserted into the body 11 via the opening 14 such that the protrusion of the acoustic matching element 12 protrudes from the opening 15. At this time, the medium supplier 19 is inserted between the body 11 and the acoustic matching element 12. Each of the two sheets of medium suppliers 19 is rolled around the core portion 19a.

Finally, as shown in FIG. 11, each of the rolled-up medium suppliers 19 is inserted into the body 11 via a gap between the body 11 and the acoustic matching element 12, thereby completing the assembly of the attachment 10.

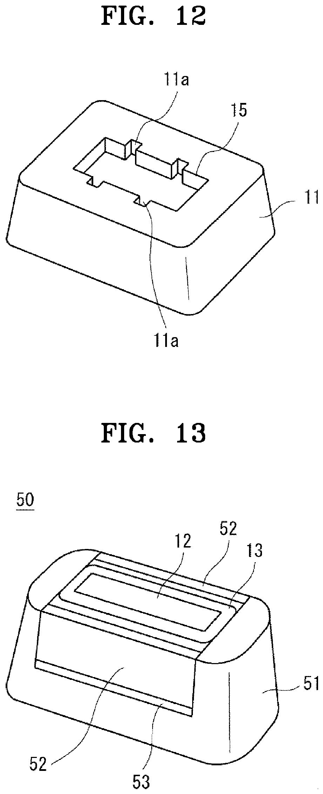

FIG. 12 illustrates another example of the body 11 according to the present embodiment 1. FIG. 12 illustrates the exterior of the body 11 when a surface of the body 11 facing an inspection target faces upwards. The opening 15 of the body 11 has a plurality of grooves 11a each ranging from the inner side of the body 11 to the outer side thereof. The grooves 11a are fine flow paths of a medium, and thus the medium easily permeates from the inner side of the body 11 to the outer side thereof due to a fine pipe phenomenon.

The body 11 may be constructed to be splittable into two or more parts. To prevent the body 11 from escaping from the probe 20, the body 11 may include a means for fitting the body 11 onto the probe 20 or a means such as a hook for enabling the body 11 to be hung up on the probe 20. The body 11 includes a fixer formed of a deformable material, such as a sponge or rubber, so that the body 11 may be stabilized without being shaken when being mounted on the probe 20 and may be mounted on any type of probe 20 without depending on the shape of the probe 20.

The acoustic matching element 12 may be formed of a material other than polymer gel. For example, the acoustic matching element 12 may be formed of a material having a good ultrasonic transmission and a low attenuation so as to maintain a shape. A water bag may be used as the acoustic matching element 12.

A thin film or the like may be further provided on a surface of the acoustic matching element 12, and thus a medium supplier may supply a medium between the thin film and the inspection target.

As described above, the attachment 10 for ultrasound probe according to the present embodiment 1 is disposed on the transceiver 21 of the ultrasound probe 20, and includes the acoustic matching element 12 which is deformed based on a surface of the inspection target 30 when the ultrasound probe 20 scans the surface of the inspection target 30, the medium supplier 13 which is near the acoustic matching element 12 and supplies the medium 16 between the inspection target 30 and the acoustic matching element 12, based on deformation of the acoustic matching element 12, and the body 11 which fixes the acoustic matching element 12 and the medium supplier 13 to the ultrasound probe 20.

In the attachment 10 for ultrasound probe according to the present embodiment 1, the body 11 accommodates the acoustic matching element 12 and the medium supplier 13 and has the opening 15 in the surface of the body 11 facing the inspection target 30, and the portions of the acoustic matching element 12 and the medium supplier 13 may protrude from the opening 15.

In the attachment 10 for ultrasound probe according to the present embodiment 1, the acoustic matching element 12 has a convex shape having a protrusion, and the protrusion may protrude from the opening 15.

In the attachment 10 for ultrasound probe according to the present embodiment 1, the acoustic matching element 13 has a 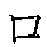 -character shape or a ⊐-character shape, and may surround at least some of the lateral surfaces of the protrusion.

In the attachment 10 for ultrasound probe according to the present embodiment 1, the opening 15 may have the grooves 11a each ranging from the inner side of the body 11 to the outer side thereof and serving as a flow path of the medium 16.

In the attachment 10 for ultrasound probe according to the present embodiment 1, the body 11 may be constructed to be splittable.

In the attachment 10 for ultrasound probe according to the present embodiment 1, the body 11 may include a deformable fixer that is disposed between the ultrasound probe 20 and the body 11 and fixes the ultrasound probe 20 to the body 11.

In the attachment 10 for ultrasound probe according to the present embodiment 1, the acoustic matching element 12 may have a hydrophilic property, and the medium 16 may be liquid including water.

In the attachment 10 for ultrasound probe according to the present embodiment 1, the acoustic matching element 12 may include polymer gel.

In the attachment 10 for ultrasound probe according to the present embodiment 1, the acoustic matching element 12 may be a water bag.

In the attachment 10 for ultrasound probe according to the present embodiment 1, the medium 16 may be water or hyaluronic acid.

In the attachment 10 for ultrasound probe according to the present embodiment 1, the medium supplier 13 may be pressed due to deformation of the acoustic matching element 12 and thus may supply the medium 16.

In the attachment 10 for ultrasound probe according to the present embodiment 1, the medium supplier 13 may be provided in contact with the acoustic matching element 12 and may be directly pressed by the acoustic matching element 12 due to deformation of the acoustic matching element 12.

In the attachment 10 for ultrasound probe according to the present embodiment 1, the medium supplier 13 may include non-woven fabric.

The ultrasound inspecting apparatus according to the present embodiment 1 may include the attachment 10 for ultrasound probe and the ultrasound probe 20.

In the ultrasound inspecting method according to the present embodiment 1, the acoustic matching element 12 and the medium supplier 13 are fixed onto the ultrasound probe 20, the acoustic matching element 12 is deformed based on a surface of the inspection target 30 when the ultrasound probe 20 scans the surface of the inspection target 30, the medium supplier 13 supplies the medium 16 between the inspection target 30 and the acoustic matching element 12, based on the deformation of the acoustic matching element 12, and the ultrasound probe 20 transmits ultrasound waves to the inspection target 30 and receives reflected waves of the ultrasound waves from the inspection target 30.

### <Embodiment 2>

An attachment for ultrasound probe according to the present embodiment 2 will now be described with reference to the attached drawings.

The attachment according to the present embodiment 2 includes a means for actively pressing a medium supplier, in addition to the components of the attachment according to embodiment 1, and uses the means as a supply increaser to supply a larger amount of medium.

FIG. 13 illustrates the exterior of an attachment 50 according to the present embodiment 2. FIG. 13 illustrates the exterior of the attachment 50 when a surface of the attachment 50 facing an inspection target faces upwards. The attachment 50 is the same as the attachment 10 according to embodiment 1 in that it includes a body 51, an acoustic matching element 12 partially exposed via an approximately center portion of a surface of the body 51 facing the inspection target, and a medium supplier 13 partially exposed via a portion of the surface of the body 51 around the acoustic matching element 12. The attachment 50 is different from the attachment 10 according to embodiment 1 in that a pressure plate 52 serving as a supply increaser is provided on a lateral surface of the body 51, namely, on a surface of the body 51 that meets with the surface of the body 51 facing the inspection target, via a hinge 53.

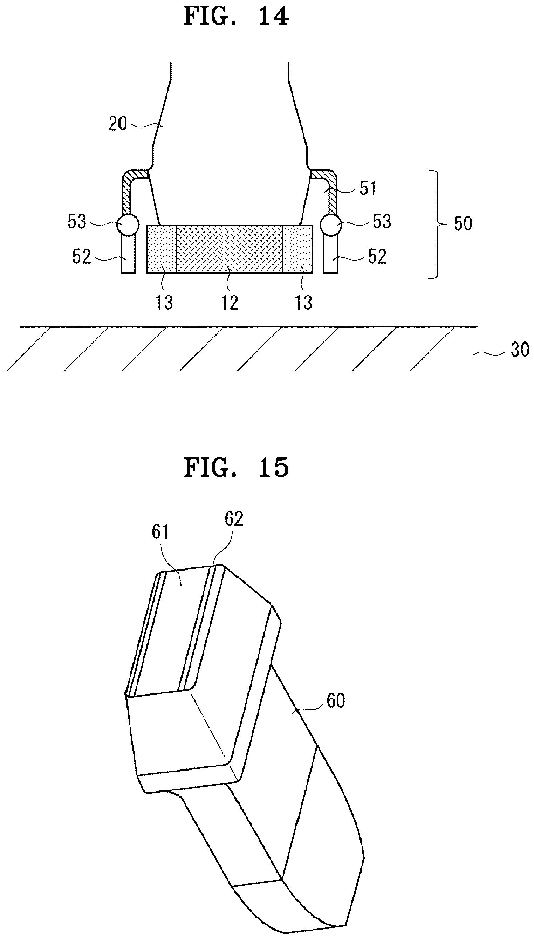

FIG. 14 is a cross-sectional view illustrating a schematic structure of the attachment 50 according to the present embodiment 2 mounted on the probe 20. The pressure plate 52 is coupled to the body 51 via the hinge 53. The present embodiment 2 is the same as the embodiment 1 in that the probe 20 scans the inspection target 30, the acoustic matching element 12 is deformed, and the medium supplier 13 is pressed by the deformed acoustic matching element 12 and thus supplies a medium.

According to the present embodiment 2, an operator presses the medium supplier 13 by pushing the pressure plate 52 inwards with his or her fingers when continuously scanning the inspection target 30, thereby supplying a larger amount of medium from the medium supplier 13.

If the acoustic matching element 12 uses polymer gel and scanning is performed for a long time, the surface of the polymer gel may become dry and wettability or lubricity may disappear, and it may be difficult to propagate ultrasound. Thus, by forcibly supplying the medium from the medium supplier 13 by using the pressure plate 52, the surface of the polymer gel is securely maintained wet, and a lubrication space between the probe 20 and the inspection target 30 is securely formed.

As described above, the attachment 50 for ultrasound probe according to the present embodiment 2 may further include the pressure plate 52, which is a supply increaser for increasing supply of the medium 16 from the medium supplier 13.

### <Embodiment 3>

An ultrasound probe according to the present embodiment 3 will now be described with reference to the attached drawings.

The ultrasound probe according to the present embodiment 3 is an integration of the attachment 10 and the probe 20 according to embodiment 1. The ultrasound probe according to the present embodiment 3 is obtained by using an acoustic lens formed of polymer gel instead of using the acoustic matching element 12 and accommodating the acoustic lens and a medium supplier within the probe.

FIG. 15 illustrates the exterior of a probe 60 according to the present embodiment 3. An acoustic lens 61 and a medium supplier 62 are exposed via a surface of the probe 60 facing an inspection target. The acoustic lens 61 is formed of polymer gel or the like that is deformable by a pressure, and the medium supplier 62 is formed of non-woven fabric or the like from which a medium may ooze out.

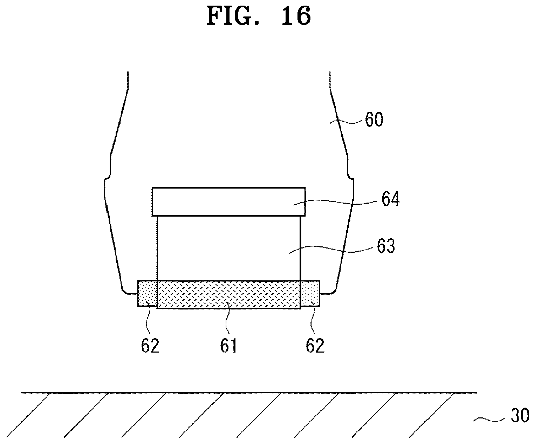

FIG. 16 is a cross-sectional view illustrating a schematic structure of the probe 60 according to the present embodiment 3. FIG. 16 illustrates a schematic structure of the probe 60 when the surface of the probe 60 facing the inspection target 30 faces down and the probe 60 is viewed from the side. The probe 60 includes the acoustic lens 61 which constitutes a portion of the surface of the probe 60 facing the inspection target 30 and converges ultrasound by using the curve of the acoustic lens 61, an ultrasound oscillator 63 which transmits or receives ultrasound, and a backing member 64 which reflects, attenuates, and absorbs ultrasound emitted backwards from the ultrasound oscillator 63. The acoustic lens 61, the ultrasound oscillator 63, and the backing member 64 are disposed within the probe 60 in this stated order starting from the surface of the probe 60 facing the inspection target 30. The medium supplier 62 which constitutes a portion of the surface of the probe 60 facing the inspection target 30 and supplies a medium is disposed around the acoustic lens 61.

The acoustic lens 61 may have a function as an ultrasound matching layer. In this case, the acoustic impedance of the acoustic lens 61 gradually changes in a thickness direction of the acoustic lens 61. Of course, the acoustic lens 61 may have only a lens function, and a special ultrasound matching layer may be provided between the acoustic lens 61 and the ultrasound oscillator 63.

Operations of the acoustic lens 61 and the medium supplier 62 when the probe 60 scans a surface of the inspection target 30 is almost the same as operations of the acoustic matching element 12 and the medium supplier 13 according to embodiment 1. The acoustic lens 61 contacts the surface of the inspection target 30 and is thus deformed, and presses the medium supplier 62. The medium supplier 62 oozes out the medium and supplies the medium between the surface of the inspection target 30 and the acoustic lens 61. Accordingly, the surface of the acoustic lens 61 is maintained wet, and a lubrication space between the ultrasound oscillator 63 and the inspection target 30 is formed, whereby an ultrasound inspection is favorably conducted.

Also in the probe 60 according to the present embodiment 3, a thin film may be further provided on the surface of the acoustic lens 61, and thus the medium may be supplied between the surface of the inspection target 30 and the thin film.

As described above, the ultrasound probe 60 according to the present embodiment 3 includes the acoustic lens 61 which is deformed based on a surface of the inspection target 30 when the ultrasound probe 60 scans the surface of the inspection target 30, and the medium supplier 62 which is near the acoustic lens 61 and supplies the medium between the ultrasound probe 60 and the inspection target 30, based on deformation of the acoustic lens 61.

In the ultrasound probe 60 for ultrasound probe according to the present embodiment 3, the acoustic lens 61 and the medium supplier 62 are provided in the ultrasound probe 60 such that they constitute a portion of a surface of the ultrasound probe 60, and the medium supplier 62 may supply a medium between the acoustic lens 61 and the inspection target 30.

An ultrasound inspecting apparatus according to the present embodiment 3 may include the above-described ultrasound probe 60.

Although an attachment for ultrasound probe, an ultrasound probe, an ultrasound inspecting apparatus, and an ultrasound inspecting method according to the present invention have been described with reference to the embodiments illustrated in the drawings in order to facilitate understanding of the present invention, the illustrated embodiments are only examples, and various modifications to the illustrated embodiments and other equivalent embodiments may be possible. Therefore, the scope of the present invention should be determined by the accompanying claims.

## Claims

1. An attachment (10) for an ultrasound probe (20), the attachment (20) comprising:
an acoustic matching element (12) provided on a transceiver (21) of the ultrasound probe (20) and deformed based on a surface of an inspection target (30) when the ultrasound probe (20) scans the surface of the inspection target (20);
a medium supplier (13) provided near the acoustic matching element (12) and configured to provide a medium (16) between the inspection target (30) and the acoustic matching element (12), based on the deformation of the acoustic matching element (12); and
a body (11) configured to fix the acoustic matching element (12) and the medium supplier (13) to the ultrasound probe (20);
wherein the body (11) accommodates the acoustic matching element (12) and the medium supplier (13) and has an opening (15) in a surface thereof facing the inspection target (30), portions of the acoustic matching element (12) and the medium supplier (13) protrude from the opening (15);
**characterized in that**
the medium supplier (13) surrounds at least some of lateral surfaces of the protruded portion of the acoustic matching element (12), wherein the lateral surfaces are surfaces of the acoustic matching element (12) meeting with the surface of the acoustic matching element (12) facing the inspection target.

2. The attachment (10) of claim 1, wherein
the acoustic matching element (12) has a convex shape having a protrusion, and
the protrusion protrudes from the opening (15).

3. The attachment (10) of claim 2, wherein the opening (15) has a groove (11a) that ranges from an inner side of the body (11) to an outer side of the body (11) and serves as a flow path of the medium (16).

4. The attachment (10) of any one of claims 1 through 3, wherein the body (11) further comprises a deformable fixer that is between the ultrasound probe (20) and the body (11) and fixes the ultrasound probe (20) to the body (11).

5. The attachment (10) of any one of claims 1 through 4, wherein the acoustic matching element (12) has a hydrophilic property, and the medium (16) is liquid including water.

6. The attachment (10) of any one of claims 1 through 4, wherein the acoustic matching element (12) comprises polymer gel.

7. The attachment (10) of any one of claims 1 through 4, wherein the acoustic matching element(12) is a water bag that accommodates water.

8. The attachment (10) of any one of claims 1 through 4, wherein the medium (16) is water or hyaluronic acid.

9. The attachment (10) of any one of claims 1 through 8, wherein the medium supplier (13) is pressed due to the deformation of the acoustic matching element (12) and supplies the medium (16).

10. The attachment (10) of any one of claims 1 through 9, wherein the medium supplier (13) is in contact with the acoustic matching element (12) and is directly pressed by the acoustic matching element (12) due to the deformation of the acoustic matching element (12).

11. The attachment (10) of any one of claims 1 through 10, wherein the medium supplier (62) includes non-woven fabric.

12. The attachment of any one of claims 1 through 11, further comprising a supply increaser that comprises a pressure plate (52) which is coupled to the body via a hinge (53) and is pushable by an operator to press the medium supplier.

13. An ultrasound inspecting apparatus, **characterized in that** the ultrasound inspecting apparatus comprises
an attachment (10) for ultrasound probes (20) of any one of claims 1 through 12; and
an ultrasound probe (20) on which the attachment (10) for ultrasound probes (20) is mounted.

14. An ultrasound inspecting method comprising
fixing an acoustic matching element (12) and a medium supplier (13) onto an ultrasound probe (20) by using a body (11), the body (11) having an opening (15) in a surface thereof facing the inspection target (30), portions of the acoustic matching element (12) and the medium supplier (13) protruding from the opening (15), wherein the medium supplier (13) surrounds at least some of lateral surfaces of the protruded portion of the acoustic matching element (12) and wherein the lateral surfaces are surfaces of the acoustic matching element (12) meeting with the surface of the acoustic matching element (12) facing the inspection target, and the acoustic matching element (12) being deformed (S30) based on a surface of an inspection target (30) when the ultrasound probe (20) scans the surface of the inspection target (30);
supplying (S50) a medium (16) between the inspection target (30) and the acoustic matching element (12), based on the deformation of the acoustic matching element (12), wherein the supplying (S50) is performed by the medium supplier (13); and
transmitting ultrasound waves (S70) to the inspection target (30) and receiving reflected waves of the ultrasound waves (S70) from the inspection target (30), wherein the transmitting and the receiving are performed by the ultrasound probe (20).

## Patentansprüche

1. Ein Anbau (10) für eine Ultraschallsonde (20), wobei der Anbau (20) aufweist:
ein akustisches Anpassungselement (12), das auf einem Transceiver (21) der Ultraschallsonde (20) bereitgestellt wird und basierend auf einer Oberfläche eines Prüfziels (30) verformt wird, wenn die Ultraschallsonde (20) die Oberfläche des Prüfziels (20) abtastet;
eine Mediumzufuhrvorrichtung (13), die nahe dem akustischen Anpassungselement (12) bereitgestellt wird und konfiguriert ist, um ein Medium (16) zwischen dem Prüfziel (30) und dem akustischen Anpassungselement (12) basierend auf der Verformung des akustischen Anpassungselements (12) bereitzustellen; und
einen Körper (11), der konfiguriert ist, um das akustische Anpassungselement (12) und die Mediumzufuhrvorrichtung (13) an der Ultraschallsonde (20) zu befestigen;
wobei der Körper (11) das akustische Anpassungselement (12) und die Mediumzufuhrvorrichtung (13) aufnimmt und eine Öffnung (15) in einer Oberfläche davon, die dem Prüfziel (30) zugewandt ist, aufweist, wobei Abschnitte des akustischen Anpassungselements (12) und der Mediumzufuhrvorrichtung (13) aus der Öffnung (15) vorstehen;
**dadurch gekennzeichnet, dass**
die Mediumzufuhrvorrichtung (13) zumindest einige von Seitenflächen des vorstehenden Abschnitts des akustischen Anpassungselements (12) umgibt,
wobei die Seitenflächen Oberflächen des akustischen Anpassungselements (12) sind, die auf die Oberfläche des akustischen Anpassungselements (12), die dem Prüfziel zugewandt ist, treffen.

2. Der Anbau (10) nach Anspruch 1, wobei
das akustische Anpassungselement (12) eine konvexe Form, die einen Vorsprung aufweist, aufweist, und
der Vorsprung aus der Öffnung (15) vorsteht.

3. Der Anbau (10) nach Anspruch 2, wobei die Öffnung (15) eine Nut (11a), die von einer Innenseite des Körpers (11) zu einer Außenseite des Körpers (11) reicht und als Strömungspfad des Mediums (16) dient, aufweist.

4. Der Anbau (10) nach einem der Ansprüche 1 bis 3, wobei der Körper (11) ferner eine verformbare Befestigung, die zwischen der Ultraschallsonde (20) und dem Körper (11) ist und die Ultraschallsonde (20) am Körper (11) befestigt, aufweist.

5. Der Anbau (10) nach einem der Ansprüche 1 bis 4, wobei das akustische Anpassungselement (12) eine hydrophile Eigenschaft besitzt und das Medium (16) Flüssigkeit, die Wasser enthält, ist.

6. Der Anbau (10) nach einem der Ansprüche 1 bis 4, wobei das akustische Anpassungselement (12) Polymergel aufweist.

7. Der Anbau (10) nach einem der Ansprüche 1 bis 4, wobei das akustische Anpassungselement (12) ein Wasserbeutel, der Wasser aufnimmt, ist.

8. Der Anbau (10) nach einem der Ansprüche 1 bis 4, wobei das Medium (16) Hyaluronsäure ist.

9. Der Anbau (10) nach einem der Ansprüche 1 bis 8, wobei die Mediumzufuhrvorrichtung (13) aufgrund der Verformung des akustischen Anpassungselements (12) gedrückt wird und das Medium (16) zuführt.

10. Der Anbau (10) nach einem der Ansprüche 1 bis 9, wobei die Mediumzufuhrvorrichtung (13) mit dem akustischen Anpassungselement (12) in Kontakt steht und aufgrund der Verformung des akustischen Anpassungselements (12) direkt vom akustischen Anpassungselement (12) gedrückt wird.

11. Der Anbau (10) nach einem der Ansprüche 1 bis 10, wobei die Mediumzufuhrvorrichtung (62) Vliesstoff aufweist.

12. Der Anbau (10) nach einem der Ansprüche 1 bis 11, ferner aufweisend eine Zufuhrerhöhungsvorrichtung, die eine Druckplatte (52) aufweist, die über ein Gelenk (53) mit dem Körper verkoppelt ist und durch eine Bedienungsperson geschoben werden kann, um auf die Mediumzufuhrvorrichtung zu drücken.

13. Eine Ultraschallprüfvorrichtung, **dadurch gekennzeichnet, dass** die Ultraschallprüfvorrichtung aufweist
einen Anbau (10) für Ultraschallsonden (20) nach einem der Ansprüche 1 bis 12; und
eine Ultraschallsonde (20), auf der der Anbau (10) für Ultraschallsonden (20) angebracht ist.

14. Ein Ultraschallprüfverfahren, aufweisend
Befestigen eines akustischen Anpassungselements (12) und einer Mediumzufuhrvorrichtung (13) auf einer Ultraschallsonde (20) mittels eines Körpers (11), wobei der Körper (11) eine Öffnung (15) in einer Oberfläche davon, die dem Prüfziel (30) zugewandt ist, aufweist, wobei Abschnitte des akustischen Anpassungselements (12) und der Mediumzufuhrvorrichtung (13) aus der Öffnung (15) vorstehen, wobei die Mediumzufuhrvorrichtung (13) zumindest einige von Seitenflächen des vorstehenden Abschnitts des akustischen Anpassungselements (12) umgibt, und wobei die Seitenflächen Oberflächen des akustischen Anpassungselements (12) sind, die auf die Oberfläche des akustischen Anpassungselements (12), die dem Prüfziel zugewandt ist, treffen, und wobei das akustische Anpassungselement (12) basierend auf einer Oberfläche des Prüfziels (30) verformt wird (S30), wenn die Ultraschallsonde (20) die Oberfläche des Prüfziels (30) abtastet; Zuführen (S50) eines Mediums (16) zwischen dem Prüfziel (30) und dem akustischen Anpassungselement (12) basierend auf der Verformung des akustischen Anpassungselements (12), wobei das Zuführen (S50) durch die Mediumzufuhrvorrichtung (13) durchgeführt wird; und
Senden von Ultraschallwellen (S70) zum Prüfziel (30) und Empfangen von reflektierten Wellen der Ultraschallwellen (S70) vom Prüfziel (30), wobei das Senden und das Empfangen von der Ultraschallsonde (20) durchgeführt werden.

## Revendications

1. Une attache (10) pour une sonde ultrasonore (20), l'attache (20) comprenant :
un élément d'adaptation acoustique (12) prévu sur un émetteur-récepteur (21) de la sonde ultrasonore (20) et déformé sur la base d'une surface d'une cible d'inspection (30) lorsque la sonde ultrasonore (20) balaie la surface de la cible d'inspection (20) ;
un dispositif d'alimentation en milieu (13) prévu à proximité de l'élément d'adaptation acoustique (12) et configuré pour fournir un milieu (16) entre la cible d'inspection (30) et l'élément d'adaptation acoustique (12) sur la base de la déformation de l'élément d'adaptation acoustique (12) ; et
un corps (11) configuré pour fixer l'élément d'adaptation acoustique (12) et le dispositif d'alimentation en milieu (13) à la sonde ultrasonore (20) ;
le corps (11) recevant l'élément d'adaptation acoustique (12) et le dispositif d'alimentation en milieu (13) et ayant une ouverture (15) située dans une surface de celui-ci faisant face à la cible d'inspection (30), des parties de l'élément d'adaptation acoustique (12) et du dispositif d'alimentation en milieu (13) faisant saillie de l'ouverture (15) ;
**caractérisée en ce que**
le dispositif d'alimentation en milieu (13) entoure au moins certaines de surfaces latérales de la partie saillante de l'élément d'adaptation acoustique (12), les surfaces latérales étant des surfaces de l'élément d'adaptation acoustique (12) qui rencontrent la surface de l'élément d'adaptation acoustique (12) faisant face à la cible d'inspection.

2. L'attache (10) selon la revendication 1, dans laquelle
l'élément d'adaptation acoustique (12) a une forme convexe ayant une saillie, et la saillie fait saillie à partir de l'ouverture (15).

3. L'attache (10) selon la revendication 2, dans laquelle l'ouverture (15) a une rainure (11a) qui s'étend d'un côté intérieur du corps (11) à un côté extérieur du corps (11) et sert de trajet d'écoulement du milieu (16).

4. L'attache (10) selon l'une des revendications 1 à 3, dans laquelle le corps (11) comprend en outre une fixation déformable qui se trouve entre la sonde ultrasonore (20) et le corps (11) et fixe la sonde ultrasonore (20) au corps (11).

5. L'attache (10) selon l'une des revendications 1 à 4, dans laquelle l'élément d'adaptation acoustique (12) présente une propriété hydrophile, et le milieu (16) est un liquide comprenant de l'eau.

6. L'attache (10) selon l'une des revendications 1 à 4, dans laquelle l'élément d'adaptation acoustique (12) comprend du gel polymère.

7. L'attache (10) selon l'une des revendications 1 à 4, dans laquelle l'élément d'adaptation acoustique (12) est une poche d'eau accueillant de l'eau.

8. L'attache (10) selon l'une des revendications 1 à 4, dans laquelle le milieu (16) est de l'eau ou de l'acide hyaluronique.

9. L'attache (10) selon l'une des revendications 1 à 8, dans laquelle le dispositif d'alimentation en milieu (13) est pressé en raison de la déformation de l'élément d'adaptation acoustique (12) et fournit le milieu (16).

10. L'attache (10) selon l'une des revendications 1 à 9, dans laquelle le dispositif d'alimentation en milieu (13) est en contact avec l'élément d'adaptation acoustique (12) et est directement pressé par l'élément d'adaptation acoustique (12) en raison de la déformation de l'élément d'adaptation acoustique (12).

11. L'attache (10) selon l'une des revendications 1 à 10, dans laquelle le dispositif d'alimentation en milieu (62) comprend du tissu non-tissé.

12. L'attache selon l'une des revendications 1 à 11, comprenant un dispositif d'augmentation de l'alimentation qui comprend une plaque de pression (52) qui est couplée au corps via une charnière (53) et peut être poussée par un opérateur afin de presser le dispositif d'alimentation en milieu.

13. Un appareil d'inspection ultrasonore, **caractérisé en ce que** l'appareil d'inspection ultrasonore comprend
une attache (10) pour sondes ultrasonores (20) selon l'une des revendications 1 à 12 ; et
une sonde ultrasonore (20) sur laquelle est montée l'attache (10) pour sondes ultrasonores (20).

14. Un procédé d'inspection ultrasonore, comprenant
fixer un élément d'adaptation acoustique (12) et un dispositif d'alimentation en milieu (13) sur une sonde ultrasonore (20) en utilisant un corps (11), le corps (11) ayant une ouverture (15) située dans une surface de celui-ci faisant face à la cible d'inspection (30), des parties de l'élément d'adaptation acoustique (12) et du dispositif d'alimentation en milieu (13) faisant saillie de l'ouverture (15), le dispositif d'alimentation en milieu (13) entourant au moins certaines de surfaces latérales de la partie saillante de l'élément d'adaptation acoustique (12), et les surfaces latérales étant des surfaces de l'élément d'adaptation acoustique (12) qui rencontrent la surface de l'élément d'adaptation acoustique (12) faisant face à la cible d'inspection, et l'élément d'adaptation acoustique (12) étant déformé (S30) sur la base d'une surface d'une cible d'inspection (30) lorsque la sonde ultrasonore (20) balaie la surface de la cible d'inspection (30) ;
fournir (S50) un milieu (16) entre la cible d'inspection (30) et l'élément d'adaptation acoustique (12) sur la base de la déformation de l'élément d'adaptation acoustique (12), l'alimentation (S50) étant effectuée par le dispositif d'alimentation en milieu (13) ; et
émettre des ondes ultrasonores (S70) à la cible d'inspection (30) et recevoir des ondes réfléchies des ondes ultrasonores (S70) de la cible d'inspection (30), l'émission et la réception étant effectuées par la sonde ultrasonore (20).
